# FASCICULE DE BREVET EUROPEEN

(11) **EP 1 052 943 B1**
(45) Date de publication et mention de la délivrance du brevet: **05.12.2001**
(21) Numéro de dépôt: 99901081.2
(22) Date de dépôt: 03.02.1999
(51) Int. Cl.: A61B 17/064

(54) **AGRAFE DE FIXATION PROTHETIQUE RESORBABLE**
ABSORBIERBARE BEFESTIGUNGSKLEMME FÜR EINE PROTHESE
ABSORBABLE PROSTHETIC MOUNTING CLIP

(30) Priorité: 03.02.1998 FR 9801509
(43) Date de publication de la demande: 22.11.2000
(73) Titulaire: SOFRADIM PRODUCTION, 01600 Trévoux (FR); PHUSIS, 38330 Saint Ismier (FR)
(72) Inventeur: ORY, François, Régis, F-69270 Fontaines Saint Martin (FR); THERIN, Michel, F-69002 Lyon (FR); HUET-OLIVIER, Jacqueline, F-38330 Saint Ismier (FR)
(74) Mandataire: Guerre, Dominique
(86) Numéro de dépôt international: IB9900190
(87) Numéro de publication internationale: WO9939645

(56) Documents cités:
- WO-A-87/01270
- US-A- 3 716 058
- US-A- 4 696 300
- US-A- 5 203 864
- US-A- 5 320 633
- : "Römpp chemie lexikon, volume 5", 1992, GEORG THIEME VERLAG, STUTTGART-NEW YORK, STUTTGART

## Description

La présente invention concerne une agrafe de fixation prothétique en matériau biorésorbable, utile pour fixer une pièce prothétique dans le corps humain par rapport à un support anatomique.

Selon la présente invention, les termes utilisés ci-après dans la description et les revendications ont la signification suivante :
- par *"matériau biorésorbable",* on entend un matériau qui est décomposé, et/ou assimilé par et/ou dans le corps humain ou animal ;
- par *"support anatomique",* on entend des tissus humains ou animaux de soutien non minéralisés.

Conformément à la figure 17 du document US-C-5 203 864, on a décrit et proposé de manière générale une agrafe de fixation prothétique, ayant une structure monolithique, constituée par un matériau biorésorbable, comprenant :
- un élément distal, ou tige d'ancrage dans le support anatomique ;
- un élément proximal, ou barreau d'arrêt par rapport à une pièce prothétique ;
- et une tige de liaison en une seule partie, ou tirant, reliant, d'un côté la tige distale d'ancrage, en un point intermédiaire de ce dernier, et de l'autre côté le barreau proximal d'arrêt, en un point intermédiaire de ce dernier.

La tige de liaison est agencée par rapport à la tige distale d'ancrage, pour déterminer au moins deux positions relatives ou angulaires de ladite tige de liaison, par rapport à la tige distale d'ancrage, à savoir :
- une position de repos, correspondant à la position initiale (c'est-à-dire après obtention de l'agrafe, par exemple par moulage ou injection) de la tige de liaison, dans laquelle cette dernière est disposée selon une première direction, correspondant à la direction dans laquelle, par traction, l'agrafe se trouve retenue dans le support anatomique ;
- et une autre position contrainte, avec ou sans rappel élastique selon la nature du matériau plastique biorésorbable, dans laquelle la tige de liaison est repliée selon une seconde direction, contre la tige distale d'ancrage ; cette seconde direction correspond à la direction selon laquelle la tige distale d'ancrage, replié contre la tige de liaison, pénètre dans et écarte le support anatomique, pour ancrer l'agrafe dans ce dernier.

Une telle agrafe peut être mise en place dans le support anatomique avec tout dispositif conforme à celui décrit dans le document US-C-5 203 864 (cf. figure 14). Ce dispositif comporte de manière générale un trocart dans lequel une fente traversante longitudinale est ménagée, et coopérant avec un poussoir interne. La tige distale d'ancrage de l'agrafe est disposé dans le trocart, avec la tige de liaison passant au travers de la fente. En appliquant l'extrémité distale du trocart par l'extérieur et contre le support anatomique, et en poussant le poussoir interne contre la tige d'ancrage, on fait pénétrer cette dernière et la tige de liaison repliée, dans l'orifice ménagé par le trocart. Puis, lorsque la tige distale d'ancrage a pénétré dans le support anatomique, on retire le trocart.

Conformément à l'art antérieur précédemment identifié, dans la position de repos de la tige de liaison (en une seule partie), la première direction de cette dernière est perpendiculaire à la deuxième direction, par ailleurs parallèle ou confondue avec la direction ou le plan principal de la tige d'ancrage.

Une telle disposition a pour inconvénient d'appliquer à la jonction entre la tige de liaison et la tige distale d'ancrage, une sollicitation angulaire cumulée importante, puisque égale à 90°, pour passer de la position de repos à la position repliée de la tige de liaison, plus 90°, lorsque l'agrafe est ancrée dans le support anatomique, pour revenir à la position de repos, sous l'effet d'une traction appliquée à partir du barreau proximal d'arrêt, soit 180° au total.

L'agrafe étant réalisée dans un matériau plastique biorésorbable, ayant des propriétés élastiques médiocres, cette sollicitation fragilise l'agrafe au niveau de la jonction précitée, ce qui entraîne souvent sa rupture. entre la tige de liaison et la tige distale d'ancrage.

La présente invention a donc pour objet de remédier à cet inconvénient. Plus précisément, l'invention a pour objet un agencement de l'agrafe, limitant la sollicitation angulaire cumulée de cette dernière, au niveau de la jonction entre la tige de liaison et la tige distale d'ancrage.

Conformément à la présente invention, dans la position de repos de la tige de liaison, au moins une partie de cette dernière est inclinée ou oblique par rapport, et au barreau proximal d'arrêt, et à la tige distale d'ancrage.

Grâce à l'invention, pour la partie précitée de la tige de liaison, la sollicitation angulaire cumulée, calculée comme précédemment, est au maximum égale à 135°, dans le cas d'une inclinaison à 45° et d'une traction redressant complètement la tige de liaison, perpendiculairement à la tige distale d'ancrage.

Cette solution apporte également d'autres avantages inattendus.

Lorsque la partie précitée de la tige de liaison est redressée, c'est-à-dire disposée perpendiculairement à la tige distale d'ancrage, son inclinaison originelle permet de disposer d'un supplément de profondeur d'ancrage, par rapport à la distance séparant la tige distale d'ancrage et le barreau proximal d'arrêt, par exemple parallèles l'un à l'autre. Ceci permet une fixation plus efficace de l'agrafe dans le support anatomique.

Et néanmoins, l'encombrement en largeur ou hauteur de l'agrafe, c'est-à-dire la distance séparant la tige d'ancrage et le barreau d'arrêt, demeure limitée, et demeure compatible avec la section des conduits tubulaires utilisés en chirurgie, et notamment coelioscopique ou laparoscopique, par lesquels l'agrafe est engagée dans le support anatomique. Autrement dit, à profondeur d'ancrage égale, l'encombrement en largeur ou hauteur d'une agrafe selon l'invention est beaucoup moins important.

Selon un mode préféré de l'invention, un gousset est ménagé à la jonction de la tige de liaison avec la tige distale d'ancrage, en sorte que la zone de repliement, ou axe d'articulation de la tige de liaison contre la tige distale d'ancrage se trouve déplacé le long de la tige de liaison, à l'écart de la zone de jonction entre la tige distale d'ancrage et la tige de liaison.

Ceci permet de distribuer les contraintes mécaniques de flexion, extension et traction de la tige de liaison par rapport à la tige distale d'ancrage, selon un rayon de courbure plus important, ce qui limite la fragilisation du matériau biorésorbable, au niveau de la jonction entre tige de liaison et tige d'ancrage.

Ce gousset limite par ailleurs le repliement complet de la tige de liaison sur la tige distale d'ancrage, et crée un interstice suffisant entre les tiges de liaison et d'ancrage, pour favoriser l'accrochage de la tige d'ancrage dans le support anatomique, dès la première sollicitation en traction, et ainsi favoriser le retour à la position de repos, et même au-delà si la traction se poursuit.

Selon une autre caractéristique préférée de la présente invention, la tige distale d'ancrage comporte, à sa propre extrémité distale, selon la direction et dans le sens de pénétration dans le support anatomique, au moins un harpon ou redan, ayant la forme d'une saillie, et permettant d'écarter ledit support anatomique. Cette saillie s'étend, en s'écartant de la tige distale d'ancrage, de l'extrémité proximale de la tige distale d'ancrage, en direction de l'extrémité proximale de la même tige.

Lors de l'insertion de l'agrafe dans le support anatomique, ce harpon ou redan permet de transférer la zone de sollicitation en flexion de la tige de liaison, du côté proximal de l'agrafe, à l'écart de la jonction entre la tige distale d'ancrage et la tige de liaison. Ceci a pour avantage de peu solliciter cette zone de jonction, relativement fragile comme dit précédemment.

Préférentiellement, en coopération avec le gousset précédemment décrit, la zone de sollicitation en flexion, qui se situe du côté du bord proximal et arrière du harpon ou redan, correspond précisément à la zone ou axe de repliement de la tige de liaison contre la tige distale d'ancrage.

s'agissant du matériau biorésorbable, celui-ci est de préférence choisi dans le groupe consistant en les polymères de p-dioxanone, les polyglycolides, les polyorthoesters, les polymères de triméthylène carbonate, les stéréocopolymères de l'acide L et D lactique, les homopolymères de l'acide L lactique, les copolymères de l'acide lactique et d'un comonomère compatible, tels que les dérivés d'alpha-hydroxy acides. De manière encore plus préférée, le matériau biorésorbable présente une polydispersité inférieure à 2.

L'invention sera mieux comprise par la description suivante, par référence au dessin en annexe, dans lequel :
- la **figure 1** représente une vue en perspective d'une agrafe de fixation en matériau biorésorbable, conforme à l'art antérieur ;
- la **figure 2** représente une vue en coupe transversale de l'agrafe de fixation selon figure 1, retenant une pièce prothétique contre un support anatomique, tel qu'une paroi musculaire ;
- la **figure 3** représente une vue à plat d'un premier mode d'exécution de l'agrafe biorésorbable selon l'invention ;
- les **figures 4, 5 et 6** se rapportent à un deuxième mode d'exécution d'une agrafe selon l'invention ; la figure 4 est une vue à plat, à échelle agrandie, la figure 5 une vue de côté, et la figure 6 représente un détail de la figure 4.

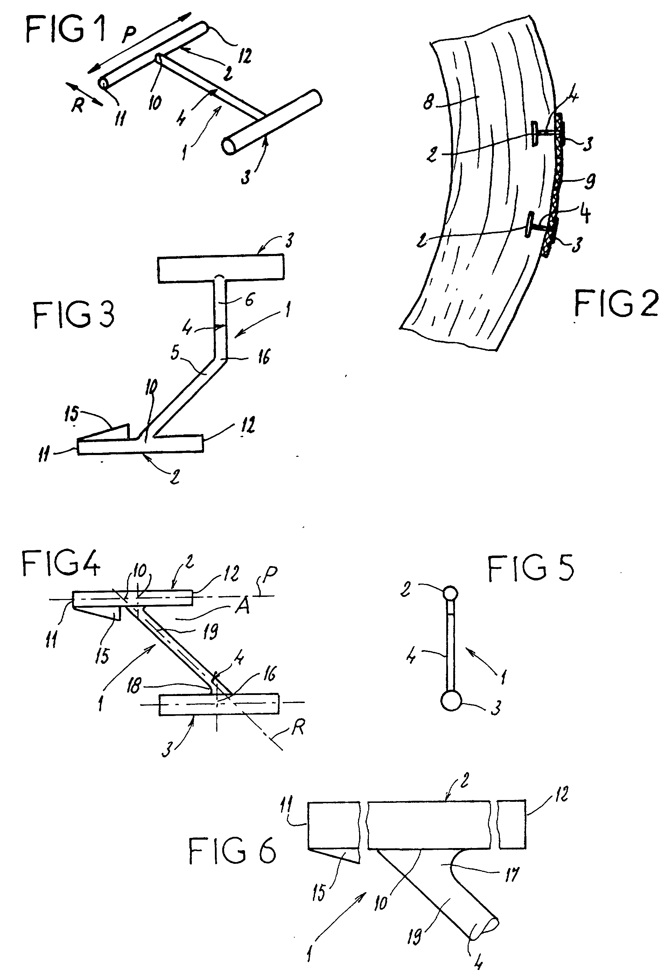

En se référant aux figures 1 et 2, une agrafe 1 de fixation prothétique selon l'art antérieur est représentée. Cette agrafe 1 présente une structure monolithique, qui peut être obtenue par exemple par moulage par injection d'un matériau biorésorbable, par exemple à base majoritaire de polymère d'acide L lactique, d'une polydispersité inférieure à 2. L'agrafe 1 comporte une tige distale d'ancrage 2, et un barreau proximal d'arrêt 3, reliés par une tige de liaison 4. La tige distale d'ancrage 2 et le barreau proximal d'arrêt 3 sont de préférence et comme représentés à la figure 1, et ont respectivement une extension transversale par rapport à la tige de liaison 4. La tige 2 et le barreau 3 s'étendent de part et d'autre de la tige de liaison 4, de manière à former un "H".

La tige d'ancrage 2 s'étend selon une première direction de pénétration P et d'écartement d'un support anatomique 8, selon laquelle ladite tige est introduite comme décrit précédemment. La tige de liaison 4 s'étend selon une deuxième direction de retenue R, selon laquelle l'agrafe 1 se trouve retenue dans le support anatomique 8, par traction à partir du barreau proximal d'arrêt 3. La tige de liaison 4 est agencée par rapport à la tige d'ancrage 2, pour avoir une position de repos dans laquelle la tige de liaison 4 est disposée selon la direction de retenue R, et une position de pénétration contrainte, repliée contre la tige d'ancrage 2, dans laquelle la tige de liaison 4 est disposée parallèlement à la direction de pénétration P. Comme décrit plus haut en se référant au brevet US-C-5 203 864, lorsque la tige de liaison 4 est repliée contre la tige d'ancrage 2, l'agrafe est alors introduite à travers la pièce prothétique 9 et dans le support anatomique 8, par l'extrémité distale 11 de la tige d'ancrage 2, par poussée sur l'extrémité proximale 12 de cette même tige 2. Lorsque la tige d'ancrage 2 a complètement pénétré dans le support, par exemple une paroi musculaire, l'angulation à la jonction 10 entre la tige d'ancrage 2 et la tige de liaison 4 agit, à la première sollicitation en traction de l'agrafe, de manière à ramener la tige de liaison 4 perpendiculairement à la tige d'ancrage 2, dans sa position de repos. L'agrafe se trouve ainsi retenue entre deux plans de fibres musculaires. En même temps, le barreau 3 d'arrêt proximal arrête le déplacement pénétrant de la tige d'ancrage 7, en venant en butée contre la pièce prothétique 9.

La figure 3 représente un premier mode d'exécution de l'agrafe selon la présente invention. Les mêmes références numériques ont été utilisées pour désigner les mêmes éléments que dans les figures 1 et 2, et seules les différences par rapport à celles-ci seront discutées.

La tige d'ancrage 2 comporte tout d'abord une saillie d'écartement 15, jouant comme dit précédemment la fonction de harpon ou redan, s'étendant en s'écartant depuis l'extrémité distale 11 en direction de l'extrémité proximale 12. Cette saillie d'écartement 15 présente une surface inclinée vers la partie proximale de l'agrafe. L'inclinaison d'une surface de la saillie 15, permet d'assurer l'écartement du support anatomique, mais également de déplacer la sollicitation en flexion exercée par le tissu prothétique 9 et la paroi musculaire 8, sur la tige de liaison 4, plus en direction du barreau proximal d'arrêt 3, c'est-à-dire plus haut sur la tige de liaison 4 telle que représentée sur la figure 3. La hausse du point de sollicitation en flexion, provoquée par la saillie, permet à la tige de liaison 4 de s'aligner de manière sensiblement parallèle à la direction de pénétration P, sans trop solliciter la jonction entre la tige d'ancrage 2 et la tige de liaison 4.

Toujours selon la figure 3, la tige 4 de liaison est inclinée dans une partie 5, par rapport à la direction de pénétration P de la tige d'ancrage 2, et par exemple à 45°. Par ailleurs, la tige 4 de liaison présente un coude 16, et s'étend dans une autre partie 6 depuis ce dernier vers le barreau d'arrêt 3, en formant un angle sensiblement droit avec celui-ci, de manière à ce que le barreau d'arrêt 3 demeure sensiblement parallèle à la tige d'ancrage 2.

Conformément aux figures 4 à 6, et selon un deuxième mode d'exécution de l'invention, l'agrafe 1 de fixation prothétique a comme précédemment une structure monolithique, constituée par un matériau plastique biorésorbable, et comprend une tige distale d'ancrage 2 dans le support anatomique 8, un barreau proximal d'arrêt 3 par rapport à la pièce prothétique 9, et une tige de liaison 4 en une seule partie reliant la tige distale d'ancrage 2 et le barreau proximal d'arrêt 3. Toujours comme précédemment, la tige de liaison 4, est agencée par rapport à la tige distale d'ancrage 2, pour déterminer au moins deux positions de cette tige de liaison 4, à savoir :
- une position de repos dans laquelle la tige de liaison 4 est disposée-selon une première direction R ;
- et une autre position contrainte, dans laquelle la tige de liaison 4 est repliée selon une seconde direction P, correspondant à la direction de pénétration dans le support anatomique 8 de la tige distale d'ancrage 2, et ce contre cette dernière.

Conformément à l'invention, dans la position de repos de la tige de liaison 4, la première direction R est inclinée par rapport à la deuxième direction P, parallèle ou identique à celle de la tige d'ancrage 2, et ceci selon un angle par exemple égal à environ 45°.

La tige de liaison 4 joint la tige distale d'ancrage 2, en un point intermédiaire 10 de cette dernière, par exemple au milieu.

Comme décrit par référence à la figure 3, la tige distale d'ancrage 2 comporte au moins une saillie 15 d'écartement, ayant la forme d'un redan ou harpon, prévue dans la direction P, s'étendant depuis l'extrémité distale 11 en direction de l'extrémité proximale 12 de la tige distale d'ancrage 2.

La tige de liaison 4 joint le barreau proximal d'arrêt 3, en un point intermédiaire 16 de ce dernier, par exemple au milieu.

Le barreau proximal 3 a une section plus importante que celle de la tige distale d'ancrage 2. Et la tige de liaison 4 a une section intermédiaire entre celles du barreau proximal d'arrêt 3, et de la tige distale d'ancrage 2 respectivement.

Dans la position de repos de la tige de liaison 4, correspondant à la configuration de l'agrafe avant son utilisation, cette tige de liaison, le barreau proximal d'arrêt 3 et la tige distale d'ancrage 2 sont disposés substantiellement dans un même plan. Le barreau d'arrêt 3 et la tige d'ancrage 2 sont disposés substantiellement parallèlement l'un à l'autre, avec la tige de liaison 4 en position inclinée ou oblique par rapport, et au barreau d'arrêt 3, et à la tige d'ancrage 2.

Comme le montre mieux la figure 6, un gousset 17 est ménagé à la jonction de la tige de liaison 4 avec la tige distale d'ancrage 2, en sorte que la zone de repliement 19, ou axe d'articulation de la tige de liaison 4 pour venir contre la tige distale d'ancrage 2, se trouve déplacé le long de cette tige 4, à l'écart de la zone de jonction 10 entre la tige distale d'ancrage 2 et la tige de liaison 4.

Un gousset 18 de même type est ménagé, du côté opposé au gousset 17, à la jonction de la tige de liaison 4 avec le barreau proximal d'arrêt 3.

### EXEMPLE :

Une agrafe conforme au mode d'exécution des figures 4 à 6 a été réalisée avec un matériau biorésorbable, comprenant un polymère d'acide L lactique, fabriqué et vendu par la Société PHUSIS sous la dénomination PHUSILINE® PLA 98, et avec le dimensionnement suivant :
- la tige d'ancrage 2 a une section de diamètre 0,9 mm, avec une longueur de 7 mm ;
- le barreau d'arrêt 3 a une section de diamètre 1,2 mm, avec une longueur de 7,2 mm ;
- la tige de liaison 4 a une section de diamètre 0,55 mm, et elle est inclinée à 45° par rapport à la tige 2 et au barreau 3 ;
- la distance séparant les axes du barreau 3 et de la tige 2 est égale à 6,5 mm.

Cette agrafe est testée par comparaison avec une agrafe témoin, en matériau plastique traditionnel, à savoir en polypropylène, conservant sensiblement les mêmes dimensions et la même forme.

Ces agrafes sont testées sur le quadriceps et le tendon rotulien d'un agneau, à savoir sur la face externe de la cuisse (tissu musculaire conventionnel avec aponévrose de couverture), et sur le tendon rotulien (tissu fibreux dense de faible épaisseur couvrant une cavité).

Des essais en traction sont effectués, en mesurant avec un tensiomètre la force nécessaire à l'arrachement de l'agrafe.

Le tableau ci-après résume les forces à l'arrachement en N.

| | | | |
|---|---|---|---|
| Agrafe selon l'invention | Cuisse externe | 13,19 | Ecart type 3,46 |
| | Tendon | 14,95 | Ecart type 2,56 |
| Agrafe témoin, en polypropylène | Cuisse externe | 14,11 | Ecart type 2,33 |
| | Tendon | 14,68 | Ecart type 3,23 |

Grâce à l'invention, et nonobstant les propriétés mécaniques limitées du matériau biorésorbable, on obtient une force à l'arrachement du même ordre que celle avec un matériau plastique traditionnel. Par ailleurs, cette force est du même ordre que celle obtenue, dans les mêmes conditions, avec des agrafes métalliques, par exemple ayant des valeurs de l'ordre de 13N.

## Revendications

1. Agrafe (1) de fixation prothétique, ayant une structure monolithique, constituée par un matériau plastique biorésorbable, comprenant une tige distale d'ancrage (2) dans un support anatomique (8), un barreau proximal d'arrêt (3) par rapport à une pièce prothétique (9), et une tige de liaison (4) disposée entre un point intermédiaire de la tige distale d'ancrage (2), et un point intermédiaire du barreau proximal d'arrêt (3), la tige de liaison (4) étant agencée par rapport à la tige distale d'ancrage (2), pour déterminer au moins deux positions de ladite tige de liaison, à savoir l'une de repos dans laquelle au moins une partie (5) de la tige de liaison (4) est disposée selon une première direction (R) par rapport à la tige distale d'ancrage (2), et l'autre contrainte dans laquelle ladite partie (5) de la tige de liaison (4) est repliée contre la tige distale d'ancrage (2) selon une seconde direction (P) et, dans la position de repos de la tige de liaison (4), le barreau proximal d'arrêt (3) et la tige distale d'ancrage (2) sont disposés dans un même plan, ledit barreau d'arrêt et ladite tige distale d'ancrage étant disposés parallèlement l'un à l'autre, **caractérisé en ce que**, dans la position de repos de la tige de liaison (4), au moins ladite partie (5) de cette dernière est en position inclinée ou oblique par rapport, et audit barreau proximal d'arrêt (3), et à ladite tige distale d'ancrage (2).

2. Agrafe (1) selon la revendication 1, **caractérisée en ce que**, dans la position de repos de la tige de liaison (4) la première direction (R) est inclinée par rapport à la seconde direction (P), parallèle à la tige distale d'ancrage (2), selon un angle égal à 45°.

3. Agrafe selon la revendication 1, **caractérisée en ce que** la tige distale d'ancrage (2) comporte au moins une saillie (15) d'écartement prévue dans ladite seconde direction (P), s'étendant depuis une extrémité distale (11) en direction d'une extrémité proximale (12) de la tige distale d'ancrage (2).

4. Agrafe selon la revendication 1, **caractérisée en ce que** le barreau proximal d'arrêt (3) a une section plus importante que celle de la tige distale d'ancrage (2).

5. Agrafe selon la revendication 4, **caractérisée en ce que** la tige de liaison (4) a une section intermédiaire entre celles du barreau proximal d'arrêt (3) et de la tige distale d'ancrage (2) respectivement.

6. Agrafe selon la revendication 1, **caractérisée en ce qu'**un gousset (17) est ménagé à la jonction de la tige de liaison (4) avec la tige distale d'ancrage (2), en sorte que la zone de repliement (19) de la tige de liaison contre la tige distale d'ancrage (2) se trouve déplacée le long de la tige de liaison (4), à l'écart de la zone de jonction (10) entre la tige distale d'ancrage (2) et la tige de liaison (4).

## Claims

1. Prosthetic mounting clip (1), having a monolithic structure, made of a biologically absorbable plastic material, comprising a distal anchoring rod (2) in an anatomical support (8), a proximal stop bar (3) relative to a prosthetic part (9), and a connecting rod (4) arranged between an intermediate point of the distal anchoring rod (2) and an intermediate point of the proximal stop bar (3), the connecting rod (4) being arranged relative to the distal anchoring rod (2), so as to determine at least two positions of the said connecting rod, namely an inoperative position in which at least a part (5) of the connecting rod (4) is arranged along a first direction (R) relative to the distal anchoring rod (2), and a stressed position in which the said part (5) of the connecting rod (4) is folded back against the distal anchoring rod (2) along a second direction (P), and, in the inoperative position of the connecting rod (4), the proximal stop bar (3) and the distal anchoring rod (2) are arranged in the same plane, the said stop bar and the said distal anchoring rod being arranged parallel to one another, **characterized in that**, in the inoperative position of the connecting rod (4), at least the said part (5) of the latter is in an inclined or oblique position relative to the said proximal stop bar (3) and to the said distal anchoring rod (2).

2. Clip (1) according to Claim 1, **characterized in that**, in the inoperative position of the connecting rod (4), the first direction (R) is inclined relative to the second direction (P), parallel to the distal anchoring rod (2), at an angle equal to 45°.

3. Clip according to Claim 1, **characterized in that** the distal anchoring rod (2) includes at least one spacing projection (15) provided in the said second direction (P), extending from a distal end (11) in the direction toward a proximal end (12) of the distal anchoring rod (2).

4. Clip according to Claim 1, **characterized in that** the proximal stop bar (3) has a greater cross section than that of the distal anchoring rod (2).

5. Clip according to Claim 4, **characterized in that** the connecting rod (4) has an intermediate cross section between those of the proximal stop bar (3) and of the distal anchoring rod (2), respectively.

6. Clip according to Claim 1, **characterized in that** a gusset (17) is formed at the junction of the connecting rod (4) with the distal anchoring rod (2), such that the zone of folding (19) of the connecting rod against the distal anchoring rod (2) is displaced along the connecting rod (4), away from the junction zone (10) between the distal anchoring rod (2) and the connecting rod (4).

## Patentansprüche

1. Prothetische Befestigungsklammer (1), mit einer monolithischen Struktur, die aus einem bioresorbierbaren Kunststoffmaterial gebildet ist, mit einem distalen Verankerungsschaft (2) zur Verankerung in einem anatomischen Träger (8), einem proximalen Sperrstab (3) zum Sperren bezüglich einem Prothesenstück (9), und einem Verbindungsschaft (4), der zwischen einer mittleren Stelle des distalen Verankerungsschaftes (2) und einer mittleren Stelle des proximalen Sperrstabs (3) angeordnet ist, wobei der Verbindungsschaft (4) bezüglich dem distalen Verankerungsschaft (2) dazu ausgestaltet ist, zumindest zwei Positionen des Verbindungsschafts zu bestimmen, nämlich eine Ruheposition, in der zumindest ein Abschnitt (5) des Verbindungsschaftes (4) gemäß einer ersten Richtung (R) bezüglich dem distalen Verankerungsschaft (2) angeordnet ist, und eine Spannposition, in der der Abschnitt (5) des Verbindungsschaftes (4) gegen den distalen Verankerungsschaft (2) gemäß einer zweiten Richtung (P) abgewinkelt ist, und wobei in der Ruheposition des Verbindungsschaftes (4) der proximale Sperrstab (3) und der distale Verankerungsschaft (2) in einer selben Ebene angeordnet sind, wobei der Sperrstab und der distale Verankerungsschaft parallel zueinander angeordnet sind, **dadurch gekennzeichnet, daß** in der Ruheposition des Verbindungsschaftes (4) zumindest der Abschnitt (5) des letzteren in einer geneigten oder schrägen Position bezüglich sowohl dem proximalen Sperrstab (3) als auch dem distalen Verankerungsschaft (2) verläuft.

2. Klammer (1) nach Anspruch 1, **dadurch gekennzeichnet, daß** in der Ruheposition des Verbindungsschaftes (4) die erste Richtung (R) bezüglich der zweiten Richtung (P), die parallel zu dem distalen Verankerungsschaft (2) verläuft, unter einem Winkel von gleich 45° geneigt ist.

3. Klammer nach Anspruch 1, **dadurch gekennzeichnet, daß** der distale Verankerungsschaft (2) zumindest einen Spreizvorsprung (15) aufweist, der in der zweiten Richtung (P) vorgesehen ist, und der sich von einem distalen Ende (11) in Richtung eines proximalen Endes (12) des distalen Verankerungsschaftes (2) erstreckt.

4. Klammer nach Anspruch 1, **dadurch gekennzeichnet, daß** der proximale Sperrstab (3) einen Querschnitt aufweist, der größer ist als derjenige des distalen Verankerungsschaftes (2).

5. Klammer nach Anspruch 4, **dadurch gekennzeichnet, daß** der Verbindungsschaft (4) einen Querschnitt aufweist, der zwischen demjenigen des proximalen Sperrstabs (3) und demjenigen des distalen Verankerungsschaftes (2) liegt.

6. Klammer nach Anspruch 1, **dadurch gekennzeichnet, daß** ein Knoten (17) an der Verbindungsstelle des Verbindungsschaftes (4) mit dem distalen Verankerungsschaft (2) ausgebildet ist, derart, daß der Bereich (19) der Abwinklung des Verbindungsschaftes gegen den distalen Verankerungsschaft (2) entlang des Verbindungsschaftes (4) von dem Verbindungsbereich (10) zwischen dem distalen Verankerungsschaft (2) und dem Verbindungsschaft (4) beabstandet verlagert ist.
